# EUROPEAN PATENT APPLICATION

(11) **EP 1 840 568 A1**
(43) Date of publication of application: **03.10.2007**
(21) Application number: 06006232.0
(22) Date of filing: 27.03.2006
(51) Int. Cl.: G01N 33/44

(54) **Apparatus and method for producing and testing of foams**

(71) Applicant: ADMATIS Kft., Partos u 16 Miskolc H-3535 (HU)
(72) Inventor: Bárczy, Pál, Dr., Miskolc H-3535 (HU); Babcsán, Norbert, Dr., Miskolc H-3530 (HU); Szöke, János, Miskolc H-3530 (HU)
(74) Representative: Emri, Jozsefné

(57) **Abstract**

The subject of the invention is a universal apparatus for production and testing of foams. The heating apparatus of the apparatus is a technological module (2) mounted on a base plate (1), turnable around its longitudinal axis, with slots (11) in its two opposite sides and equipped with a bogie (23), in the test chamber (27) of which a cartridge (4) having removable window plates (44) on its two opposite sides can be placed on the bogie (23), and a testing system consisting of ray sources and detectors fixed onto the base plate (1) in a releasable manner is arranged around the technological module (2).

A further subject of the invention is a cartridge in which foam can be generated, as well as a method with which the foam structure can be tested during foam generation and/or subsequently.

## Description

The subject of the invention is a universal apparatus and a method for the production and in-process or subsequent testing of metal, polymer and ceramic foams.
Foam products can be found in numerous places in our surroundings. The group of synthetic polyurethane foams used for impact absorption in the automotive industry is well known. The construction industry is another important field of the application of foams, as closed- and open-cell insulating plates are built-in in large quantities. The developing space industry prefers to use high-strength metal foams in order to reduce weight in its new design structures.
At present there is no universal apparatus facilitating the testing of foams that measures and records the structure of generated foams during foam generation through the single operation of several testing techniques. The biggest deficiency of the currently used apparatuses is that they are not suitable for foam generation, or they can monitor the process of foam generation only partially. The imaging techniques are not integrated into the foam generating apparatuses, only the result of foam generation, the produced foam can be tested. Another difficulty is that the known apparatuses allow the use of only one testing method at a time. For the testing of several parameters the foaming operation has to be repeated, which is costly. Usually the apparatuses cannot be automated, and their operation under special (e.g. cosmic space) conditions is also difficult.
Most of the patent applications dealing with foams or high-temperature universal apparatuses describe an apparatus or method suitable for crystallization or foam production. Only some of the applications describe a device that also measures any technical parameter of the foam during foaming or subsequently.
For example, patent application No. US 5429341 describes a universal crystallizing apparatus equipped with a heating apparatus and a capsule exchanger. The apparatus is suitable for use under cosmic space conditions, for high-temperature metal technologies, it is universal and it is equipped with an automatic capsule exchanger, but it cannot be used for performing foam testing.
According to patent application No. EP0697583 the generation and development of a foam structure above the surface of a liquid can be monitored successfully with pressure gauges and ultrasonic transmitters located in the foam domain. According to this solution pressure cells and reflective ultrasonic sensors are placed in a closed space in which the size of the foam structure generated above the liquid, that is the extent of foaming can be measured. The built-in sensors measure the height of the foam generated above the water surface, but they cannot record the foam structure. A signal amplifying and signal shaping unit is connected to the apparatus, allowing the direct evaluation of the technical data obtained from the apparatus. The deficiency of the testing apparatus is, however, that only water-based foams can be tested with this apparatus, at ambient temperature.
Patent specification No. US6807849 describes a foam generator equipped with a viscometer. According to the invention the liquid to be foamed is delivered into a thermally controllable test chamber, and propellant is added to it from a cylinder. This way a stabilized foam structure is generated, which passes through the viscometer connected to the apparatus, and its viscosity is measured. The deficiency of this testing apparatus is that the process of foam generation cannot be monitored. This solution is suitable for the rheological testing of primarily detergents and related foams (aqueous cleaning foams).
The testing of metal foams is especially difficult, as their production requires high temperatures and the required metal technology. Today metal foams can be qualified only after production, with subsequent measurements.
A heating apparatus (furnace) suitable for the production of metal foams is described in patent No. CN1488011, according to which a foamable metal applied to a polymer carrier base is heated in a tunnel furnace. In the tunnel furnace the generation of the metal foam and the separation of the plastic layer takes place in different heating zones. After production (in continuous operation) the metal foam sheet can be rolled up and removed from the furnace continuously. It is not possible to measure the foam parameters in the apparatus.
At present metal foams are qualified after production, with subsequent measurements, on the basis of their foam structure and strength. Another difficulty is that there is no know apparatus in which metal foams of appropriate quality can be produced and the necessary measurements can also be performed.
The aim of this invention is the development of a universal apparatus suitable for the production of various aqueous, polymer and metal foams, and their complex in-process (in-situ) testing.
The aim of the invention is also the development of a foam generating cartridge in which both the foaming and the testing of the foam can be implemented.
A further aim of the invention is the development of a method for the testing of foams, especially metal foams, with which not only the produced foams can be qualified, but the production process can also be monitored.
The set aim was achieved by the development of an apparatus the main parts of which include a heating apparatus, a sample holder (cartridge) and a testing system. The heating apparatus is a technological module further developed from an in itself known, modular, multi-zone electric furnace, turnable around its longitudinal axis and with slots in its two opposite sides. The cartridge can be placed on a bogie in the test chamber of the technological module. The technological module is built uniaxially onto a rotary table mounted preferably centrally on a base plate, and the testing system consisting of ray sources and detectors fixed onto the base plate in a releasable manner is arranged around it.
Foam generation takes places in the cartridge. The cartridge is a rectangular frame structure with insulation defining the foam domain, the stability of which is ensured by side frames, and a foot and a lid structured by longitudinal grooves. The openings of the two opposite largest surface sides of the cartridge are covered by removable window plates. The material of the cartridge is such that it is suitable for the use of both low- and high-temperature foam producing techniques. There are window plates on the largest surface sides of the cartridge, which can be made of transparent materials or materials suitable for the performance of reflection tests, thus glass, metal or polymer. Because of the window plates the cartridge is transparent from the point of view of the testing beam. In the frame structure there is a pipe system consisting of tanks and ducts for conducting liquids and gases, required for delivering the propellants for foam generation.
The cartridge can be moved manually, or by automatic control. For automatic operation the apparatus has program control automatics and a cartridge exchanger.
In the apparatus foams, especially metal foams can be tested with the method according to the invention in such a way that the foam generated in the cartridge is transilluminated during foam generation or subsequently with at least one ray source, or in a given case the foam sample is used as a ray source. The foam structure is imaged, the data is recorded with an imaging technique, the image material is evaluated and the foam structure is qualified.
During the test the cartridge containing the foam sample is placed on the bogie of the technological module. After fixing the required gas inlet and liquid outlet connectors, as soon as the set ambient parameters (temperature, pressure, etc.) are reached, the technological module together with the cartridge is turned into testing position by means of the rotary table. For each test the technological module is turned again so that the side slots face a ray source and a detector. The window plates of the cartridge are turned into a position perpendicular to the beam by means of the bogie.
With the cartridge and the technological module fixed in position, the foaming experiment can be started by heating the technological module to the necessary temperature with electric current, then liquid, foam or propellant is delivered into the tank of the cartridge through a propellant inlet connector, from where the foam material gets into the foam domain through an injector.
The spatial structure of the generated foam can be reconstructed more accurately by means of a series of tomographic images, for this purpose the cartridge fixed onto the bogie can be turned in both directions from the perpendicular testing position. During the taking of the series of images the position of the technological module is unchanged, however the set ambient parameters can be changed freely.
The foaming technology and its base materials shall be planned and prepared in advance in every case. Foaming takes place in the foam domain of the cartridge. Foaming can take place with or without the heating of the technological module. The imaging techniques shall be turned on before the foaming, thus the foaming process can be monitored simultaneously, with image visualization and recording, as necessary, then evaluated.

A preferred embodiment of the universal foam producing and testing apparatus according to the invention is shown in detail in the following figures:
Figure 1: An axonometric drawing of the apparatus in loading position
Figure 2: A side-view drawing of the apparatus in automatic operation
Figure 3: A sectional axonometric drawing of the technological module of the apparatus
Figure 4: An axonometric drawing of the cartridge
Figure 5: A longitudinal cross-section drawing of the cartridge

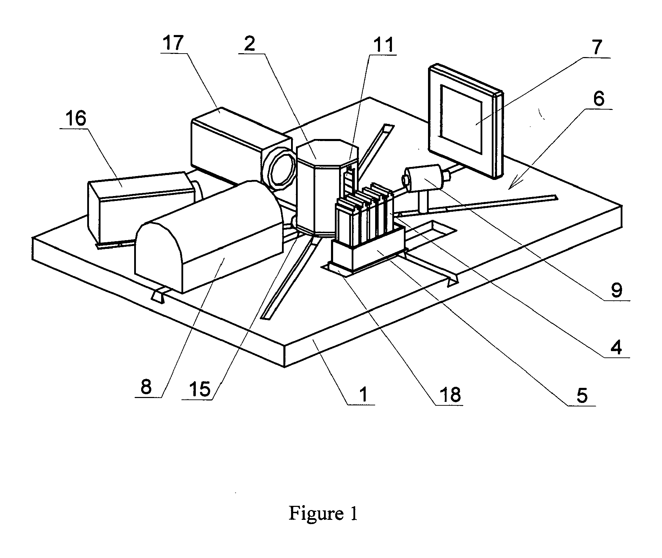

Figure 1. shows the apparatus in loading position. The technological module 2 forming the central unit of the apparatus is built uniaxially onto the rotary table 15 mounted preferably in the centre of the base plate 1. This technological module 2 is served by the cartridges 4 containing the foam material and placed in the cartridge holder 5. The base plate 1 is structured by grooves 6. The testing apparatuses and sensors are fixed in the grooves in a releasable manner, this way they are located around the technological module. E.g. the X-ray source 8 and the panel detector 7 receiving the beam 19 thereof, the Laser source 9 and the video recorder 16 or infra camera 17 receiving the signal thereof. The distance of the testing apparatuses from the technological module 2 can be adjusted as a function of the focal distances determined by the imaging techniques, as necessary, then they can be fixed in a given position in a releasable manner. The cartridge holder 5 is mounted on a transport table 18. The cartridge 4 can be pushed into the technological module 2 through the slots 11. The geometry of the slots 11 corresponds to the cross-sectional size of the cartridge 4. The technological module 2 can be turned together with the rotary table 15. The cartridge holder 5 contains the prepared cartridges 4.

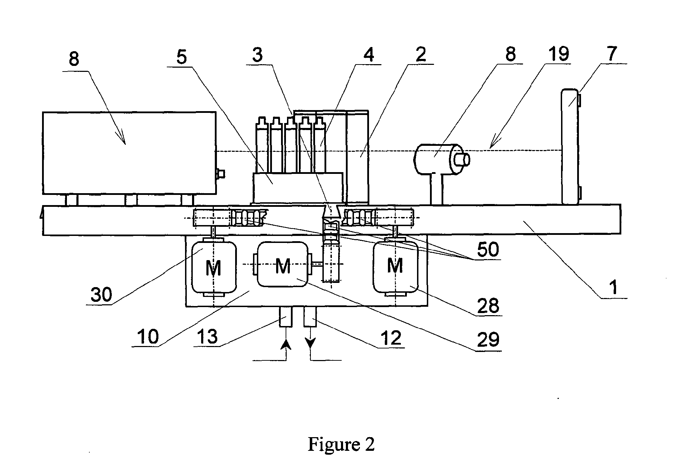

Figure 2. shows the parts required for automatic operation. There is a cartridge exchanger 10 under the base plate 1, an integral part of which is the mechanical connector 3 for the mechanical fixing of the cartridge holder 5 and the delivery and discharge of propellants and other foam materials. There is a propellant inlet 13 and a propellant outlet 12 connecting to the mechanical connector 3 for delivering the propellant required for foaming and discharging the excess gases, respectively. A transport motor 30 and a belt 50 connected to it is installed in the cartridge exchanger 10 for transporting the cartridge holder 5. An exchanger motor 29 for exchanging the cartridges, and a rotatory motor 28 for turning the technological module 2 is also installed.

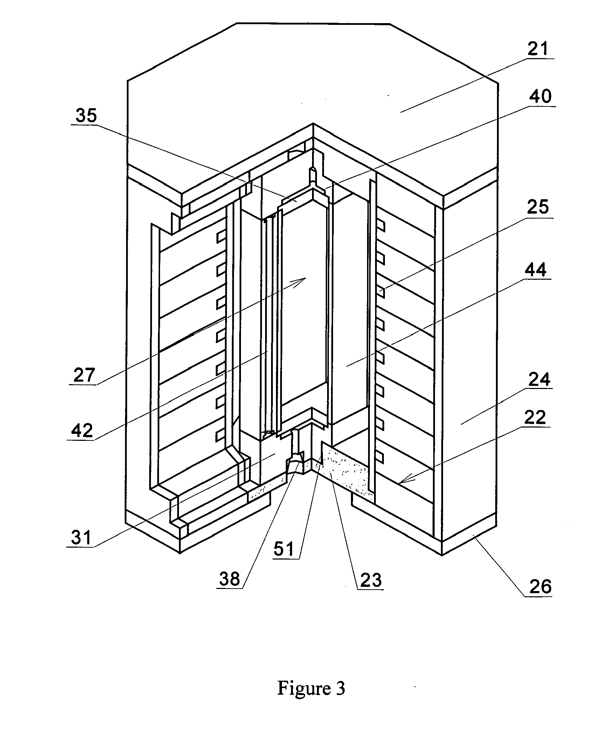

Figure 3. shows a detailed axonometric drawing of the technological module 2 together with a sectional drawing of the cartridge. The technological module 2 is a further developed version of the multi-functional crystal growing apparatus described in patent application No. P0148311, a modular, multi-zone electric furnace, in the two opposite sides of which there are slots 11 for inserting the cartridge 4. The technological module 2 is partly transparent, that is when it is placed between a ray source and a detector in the appropriate manner, the testing ray starting from the source can reach the detector unhindered through the slots in its sides. The test chamber 27 of the technological module 2 is formed by heating segments 22 built on one another, and a bogie 23 is installed in it. The cartridge 4 can be seated on the matching surface 51 of the bogie 23, and can be turned in the test chamber 27 of the technological module 2. The heating segments 22 are surrounded by a case 24, which is closed on the top by a cover plate 21 and at the bottom by a bottom plate 26. The technological module 2 works as a heating apparatus, in which the temperature field around the cartridge 4 can be regulated by computer-control. The generated heat is produced by heater-resistance wires 25.

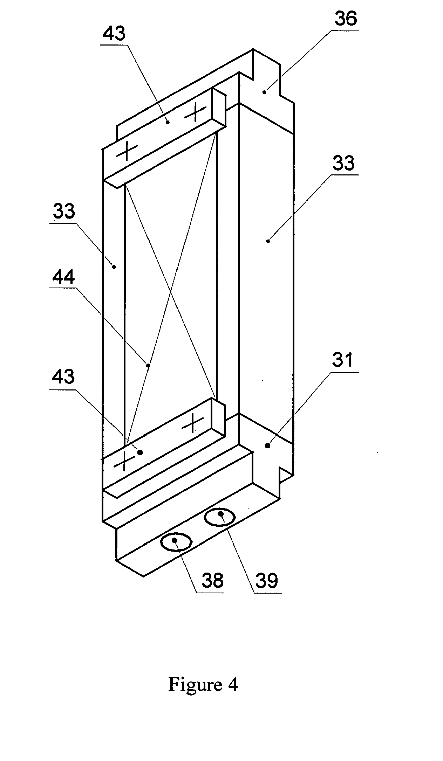

Figure 4. shows an axonometric drawing of the cartridge 4. The cartridge 4 is a rectangular body defined at the bottom by a foot 31, on the sides by 2 side frames 33, and on the top by a lid 36. The largest plane surfaces on the two opposite sides of the cartridge 4 are covered by window plates 44, the material of which can be metal, glass or ceramic, as necessary for the test. The window plates 44 ensure the transparency required by the various beams 19. Optical transparency can be ensured for example by glass or quartz window plates, while optically non-transparent window plates made of boron nitride show good X-ray transparency. There are clips 43 on the side surfaces of the side frames 33 in order to secure the window plates 44. Figure 4 shows that a foot connector 38 and a lid connector 39 connects to the propellant inlet 13 and propellant outlet 12 shown in Figure 2, respectively. The foot 31 and lid 36 are structured by longitudinal grooves ensuring the connection of the cartridge 4 to the cartridge exchanger 10, and its seating on the bogie 23.

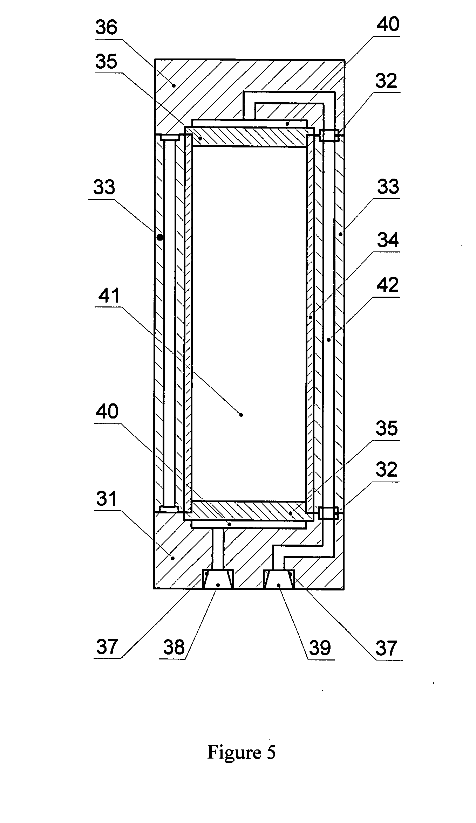

Figure 5. shows well that the foam domain 41 of the cartridge 4 is defined on the sides by side frames 33 equipped with insulation 34. The duct 42 starting from the lid connector 39 ends in the tank 40 of the lid 36. The duct 42 runs in the side frame 33, it is hermetically sealed by the installed seals 32. The junctions are sealed by means of cone seals 37. The insulation 34 around the foam domain 41 is exchangeable, thus its material can always be selected for the given experiment, so that the generated foam does not react with its surroundings. The tanks 40 ensure the storage, delivery of the liquids and gases required for the experiments into the foam domain 41. All structural elements of the foam tester are made of materials suitable for use under special conditions, even under the operating conditions found in cosmic space.

In the course of the operation of the universal foam producing and foam testing apparatus first the cartridges 4 shall be placed in the cartridge holder 5 located on the base plate 1, then the technological module 2 shall be turned into loading position, that is the slot 11 in the direction of the cartridge holder 5 either manually, or with the available automatics. In the cartridge holder 5 the connector 3 shall be fixed to the cartridge 4, then the cartridge 4 shall be pushed into the technological module 2 and seated on the bogie 23 there. This bogie 23 can be turned together with the technological module 2. When the window plate 44 of the cartridge 4 is perpendicular to the testing beam 19, the position of the cartridge 4 shall be fixed with the connector 3. Then the technological module 2 shall be turned further until the slot 11 faces the beam 19 performing the imaging. This way, actually, the independent rotation of the technological module 2 and the cartridge 4 can be implemented. By turning the technological module 2, the slot 11 can be positioned in front of a ray source, thus the performance of transmission, that is transillumination, or reflection tests becomes possible through the same slots 11 which ensure the loading of the cartridges 4.
After adjusting and fixing the cartridge 4 and the technological module 2 in the appropriate position, the foaming experiment can be started by delivering liquid, foam or propellant through the propellant inlet 13 into the tank 40 of the cartridge 4, from where the foam material gets to the foam domain through the injector 35 as a result of the overpressure. Foaming takes place in the foam domain 41. Controlled pressure conditions can be maintained on the injectors 35 located in the two ends of the cartridge through the tank 40, duct 42, lid connector 39 or foot connector 38 and connector 3 connected to them. The tank 40 facilitates the even medium resistance of the injector 35. The direction of flow is optional in both connectors, thus injection can be performed both in the foot 31 and the lid 36. Before and during foaming the technological module 2 maintains the appropriate thermal conditions.

The imaging techniques shall be turned on before the foaming, thus the whole process of foam generation can be monitored simultaneously, with image visualization and recording. Foaming can take place either with the heating of the technological module 2, primarily for the generation of metal or particle-stabilized metal foams, or without using the heating option provided by the technological module, exclusively with the foam generation option provided by the liquid or gas delivered into the foam domain 41 of the cartridge 4 through the tank 40 and the injector 35 by means of the foot connector 38 or the lid connector 39. The foam structure generated in the foam domain 41 can be recorded during foam generation by saving the image data provided by a ray source (for example the X-ray source 8) and a detector (for example the panel detector 7) with a data recorder. After image recording the technological module 2 shall be cooled. The cartridge 4 shall be removed from the technological module 2 by performing the steps described above in reverse order. Namely, after reaching a normalized temperature the technological module 2 shall be connected to the cartridge 4 again by means of the connector 3, then it shall be turned in the direction of the cartridge holder 5 by turning the technological module 2. Then the connection between the technological module 2 and the cartridge 4 through the connector 3 shall be released and the slot 11 of the technological module 2 shall also be turned in the direction of the cartridge holder 5. Then the cartridge 4 shall be pushed into the cartridge holder 5 by means of the cartridge exchanger 10. In automatic operation the cartridge holder 5 can change its place perpendicularly to the direction of pushing by means of a motor 30 so that the connector 3 matches another cartridge 4. During a repeated test from another position another cartridge 4 can be transported to the technological module 2 from the cartridge holder 5, that is the foaming experiment can be performed again in automatic operation as well.

The universal foam producing and testing apparatus according to the invention is also suitable for the complex in-process (in-situ) testing of aqueous, polymer and metal foams. The apparatus is equipped with imaging devices combining the advantages of dynamic and static tests, thus, uniquely, the technology and the final product can be characterized at the same time. In-situ transillumination and reflection tests can be performed in the apparatus at the same time or with a small time difference in such a way that neither the sources nor the detectors require positioning.

The apparatus is suitable for operation under special test conditions as well. For example, under cosmic space conditions it is especially advantageous that a series of cartridges 4 can be prepared in advance and placed in the cartridge holder 5, allowing fully automatic operation. The testing technique can be pre-selected in the control, and several cartridges can be prepared for the purpose of testing. Experimental foaming can also be performed without the operation of the imaging apparatuses, thus the method can be used for small-scale production as well.

Examples of foaming and foam testing:

### Example 1: Foam generation testing.

At the start of the experiment there is liquid in the foam domain 41, the foaming of which is performed by adding gas to it through the foot connector 38. The gas gets into the tank 40 through the duct 42, then after reaching the appropriate pressure it gets into the liquid in the foam domain 41 through the injector 35. The generated foam fills the foam domain fully, the injector 35 located in the lid 36, as a passive element, prevents the further expansion of the foam, and the excess gases can be conducted to the lid connector 39 through the duct 42 connected to it, thus ensuring closed-system operation. The process is performed with the simultaneous operation of an imaging technique and image recording. The image result is evaluated.

### Example 2: Foam generation testing.

At the start of the experiment there is a solid test piece containing a foaming reagent in the foam domain 41, foaming takes places as a result of the temperature increase provided by the technological module. The generated foam fills the foam domain partly, the injectors 35 located in the foot 31 and the lid 36, as passive elements, ensure that the excess gases are conducted to the foot connector 38 and the lid connector 39 through the duct 42, thus ensuring closed-system operation. The process is performed with the simultaneous operation of an imaging technique and image recording. The image result is evaluated.

### Example 3: Foam stability testing.

At the end of the experiments described in the previous examples, gas is conducted to both injectors 35 through the foot connector 38 and the lid connector 39, and by changing the pressure, overpressure or underpressure is created in the foam domain 41. The process is performed with the simultaneous operation of an imaging technique and image recording. The image result is evaluated.

### Example 4: Foam tomography.

At the end of the experiment described in Example 1, the fixing of the bogie 23 is released in the technological module 2 fixed in transillumination testing position, thus the cartridge 4 can be turned around its longitudinal axis. Tomographic images taken during the turning of the cartridge 4 are suitable for the reconstruction of the spatial structure of the generated foam.

### List of reference numbers:

- 1: base plate
- 2: technological module
- 3: connector
- 4: cartridge
- 5: cartridge holder
- 6: groove
- 7: panel detector
- 8: X-ray source
- 9: Laser source
- 10: cartridge exchanger
- 11: slot
- 12: propellant outlet
- 13: propellant inlet
- 15: rotary table
- 16: video recorder
- 17: infra camera
- 18: transport table
- 19: beam
- 21: cover plate
- 22: heating segment
- 23: bogie
- 24: case
- 25: heater wire
- 26: bottom plate
- 27: test chamber
- 28: rotatory motor
- 29: exchanger motor
- 30: transport motor
- 31: foot
- 32: seal
- 33: side frame
- 34: insulation
- 35: injector
- 36: lid
- 37: cone seal
- 38: foot connector
- 39: lid connector
- 40: tank
- 41: foam domain
- 42: duct
- 43: clip
- 44: window plate
- 50: belt
- 51: matching surface

## Claims

1. A universal apparatus for production and testing of foams, comprising a heating apparatus, a sample holder (cartridge) and a testing system, wherein the heating apparatus is a technological module (2) mounted on a base plate (1), turnable around its longitudinal axis, with slots (11) in its two opposite sides and equipped with a bogie (23), in the test chamber (27) of which the cartridge (4) having removable window plates (44) on its two opposite sides can be placed on the bogie (23), and the testing system consisting of ray sources and detectors fixed onto the base plate (1) in a releasable manner is arranged around the technological module (2).

2. The apparatus according to claim 1, wherein the technological module is the further developed version of an in itself known, modular, multi-zone electric furnace.

3. The apparatus according to claim 1, wherein the technological module (2) is built uniaxially onto a rotary table (15) located on the base plate (1).

4. The apparatus according to claim 1, wherein it has program control automatics and a cartridge exchanger (10).

5. The apparatus according to claim 1, wherein the geometry of the slots (11) of the technological module (2) corresponds to the cross-sectional size of the cartridge (4).

6. The apparatus according to claim 1, wherein the bogie (23) has a matching surface (51) corresponding to the geometry of the foot (31) of the cartridge (4).

7. The apparatus according to claim 1, wherein the bogie (23) and the cartridge (4) fixed onto it can be turned in the technological module (2).

8. A cartridge for the universal foam producing and testing apparatus, wherein the cartridge (4) is a rectangular frame structure with insulation (34) defining the foam domain (41), the stability of which is ensured by side frames (33), and a foot (31) and a lid (36) structured by grooves, the openings of the two opposite largest surface sides of the cartridge (4) are covered by removable window plates (44), the window plates (44) can be secured by clips (43), furthermore there are injectors (35) in the foot (31) and the lid (36), and in the frame structure there is a pipe system consisting of tanks (40) and ducts (42) for conducting liquids and gases, ending in a foot connector (38) and a lid connector (39), and hermetically sealed by means of seals (32), as well as cone seals (37) connecting to the ducts (42).

9. The cartridge for the universal foam producing and testing apparatus according to claim 8, wherein the material of the window plates (44) of the cartridge (4) is metal, polymer, glass or ceramic.

10. A method for the testing of foams, especially metal foams in the apparatus according to claim 1, wherein the foam generated in the cartridge (4) is transilluminated during foam generation and/or subsequently with at least one ray source, and/or the foam sample is used as a ray source, the foam structure is imaged, the data is recorded with an imaging technique, the image material is evaluated and the foam structure is qualified.
